# EUROPÄISCHE PATENTSCHRIFT

(11) **EP 1 725 560 B1**
(45) Veröffentlichungstag und Bekanntmachung des Hinweises auf die Patenterteilung: **07.07.2010**
(21) Anmeldenummer: 05715825.5
(22) Anmeldetag: 08.03.2005
(51) Int. Cl.: C07D 487/04, A01N 43/90

(54) **5,6-DIALKYL-7-AMINO-TRIAZOLOPYRIMIDINE, VERFAHREN ZU IHRER HERSTELLUNG UND IHRE VERWENDUNG ZUR BEKÄMPFUNG VON SCHADPILZEN SOWIE SIE ENTHALTENDE MITTEL**
5,6-DIALKYL-7-AMINO-TRIAZOLOPYRIMIDINES, METHOD FOR THEIR PRODUCTION, THEIR USE FOR CONTROLLING PATHOGENIC FUNGI AND AGENTS CONTAINING SAID COMPOUNDS
5,6-DIALKYL-7-AMINO-TRIAZOLOPYRIMIDINES, PROCEDES POUR LEUR PRODUCTION, LEUR UTILISATION POUR LUTTER CONTRE DES CHAMPIGNONS NUISIBLES, AINSI QU'AGENTS LES CONTENANT

(30) Priorität: 10.03.2004 DE 102004012018
(43) Veröffentlichungstag der Anmeldung: 29.11.2006
(73) Patentinhaber: BASF SE, 67056 Ludwigshafen (DE)
(72) Erfinder: TORMO I BLASCO, Jordi, 69514 Laudenbach (DE); BLETTNER, Carsten, 68165 Mannheim (DE); MÜLLER, Bernd, 67227 Frankenthal (DE); GEWEHR, Markus, 56288 Kastellaun (DE); GRAMMENOS, Wassilios, 67071 Ludwigshafen (DE); GROTE, Thomas, 67157 Wachenheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); WAGNER, Oliver, 67433 Neustadt (DE); NIEDENBRÜCK, Matthias, 67117 Limburgerhof (DE); SCHERER, Maria, 76829 Godramstein (DE); STRATHMANN, Siegfried, 67117 Limburgerhof (DE); SCHÖFL, Ulrich, 68782 Brühl (DE); STIERL, Reinhard, 67251 Freinsheim (DE)
(86) Internationale Anmeldenummer: PCT/EP2005/002426
(87) Internationale Veröffentlichungsnummer: WO 2005/087772

(56) Entgegenhaltungen:
- EP-A- 0 141 317
- EP-A- 0 215 382
- EP-A- 0 770 615
- WO-A-03/009687
- GB-A- 1 148 629

## Beschreibung

Die vorliegende Erfindung betrifft 5,6-Dialkyl-7-amino-triazolopyrimidine der Formel l 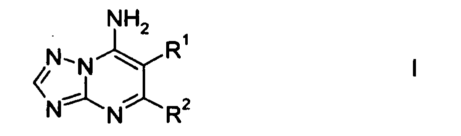 in der die Substituenten folgende Bedeutung haben:
- R¹: C₅-C₉-Alkyl oder C₃-C₁₀-Alkoxyethylen;
- R²: n-Propyl oder n-Butyl.

Außerdem betrifft die Erfindung Verfahren zur Herstellung dieser Verbindungen, sie enthaltende Mittel sowie ihre Verwendung zur Bekämpfung von pflanzenpathogenen Schadpilzen.

In GB 1 148 629 werden 5,6-Dialkyl-7-amino-triazolopyrimidine allgemein vorgeschlagen. Aus EP-A 141 317 sind einzelne fungizid wirksame 5,6-Dialkyl-7-amino-triazolopyrimidine bekannt. Ihre Wirkung ist jedoch in vielen Fällen nicht zufriedenstellend. Davon ausgehend, liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Des weiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus den oben genannten Schriften durch die spezielle Ausgestaltung des Substituenten in der 5-Position des Triazolopyrimidin-Gerüstes.

Die Verbindungen der Formel I weisen eine gegenüber den bekannten Verbindungen erhöhte Wirksamkeit gegen Schadpilze auf.

Die erfindungsgemäßen Verbindungen können auf verschiedenen Wegen erhalten werden. Vorteilhaft werden die erfindungsgemäßen Verbindungen erhalten, indem man substituierte β-Ketoestern der Formel II mit 3-Amino-1,2,4-triazol der Formel III zu 7-Hydroxytriazolopyrimidinen der Formel IV umsetzt. Die Gruppen R¹ und R² in Formeln 11 und IV haben die Bedeutungen wie für Formel I und die Gruppe R in Formel II bedeutet C₁-C₄-Alkyl, aus praktischen Gründen ist Methyl, Ethyl oder Propyl darin bevorzugt.

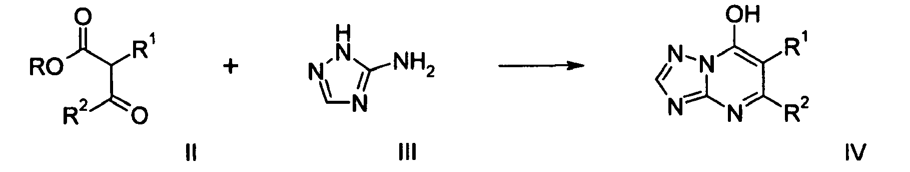

Die Umsetzung der substituierten β-Ketoester der Formel II mit den Aminoazolen der Formel III kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe, wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine und Mischungen dieser Lösungsmittel mit Wasser in Frage. Als Katalysatoren kommen Basen wie voranstehend genannt, oder Säuren, wie Sulfonsäuren oder Mineralsäuren in Frage. Besonders bevorzugt wird die Umsetzung ohne Lösungsmittel oder in Chlorbenzol, Xylol, Dimethylsulfoxid, N-Methylpyrrolidon durchgeführt. Besonders bevorzugte Basen sind tertiäre Amine wie Tri-isopropylethylamin, Tributylamin, N-Methylmorpholin oder N-Methylpiperidin. Die Temperaturen liegen zwischen 50 und 300°C, vorzugsweise bei 50 bis 180°C, wenn in Lösung gearbeitet wird [vgl. EP-A 770 615; Adv. Het. Chem. Bd. 57, S. 81ff. (1993)].

Die Basen werden im allgemeinen in katalytischen Mengen eingesetzt, sie können aber auch äquimolar, im Überschuss oder gegebenenfalls als Lösungsmittel verwendet werden.

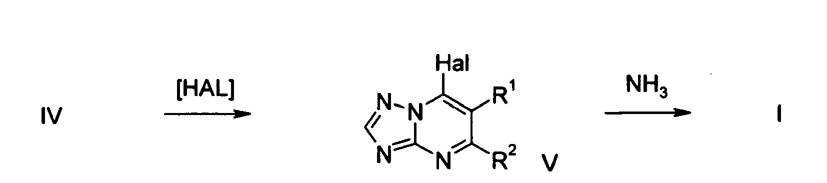

Die so erhaltenen Kondensationsprodukte der Formel IV fallen aus den Reaktionslösungen meist in reiner Form aus und werden nach dem Waschen mit dem gleichen Lösungsmittel oder mit Wasser und anschließendem Trocknen mit Halogenierungsmitteln, insbesondere Chlorierungs- oder Bromierungsmittel zu den Verbindungen der Formel V, in der Hal für Chlor oder Brom, insbesondere für Chlor steht, umgesetzt. Bevorzugt erfolgt die Umsetzung mit Chlorierungsmitteln, wie Phosphoroxychlorid, Thionylchlorid oder Sulfurylchlorid bei 50°C bis 150°C vorzugsweise in überschüssigem Phosphoroxitrichlorid bei Rückflußtemperatur. Nach dem Verdampfen des überschüssigen Phosphoroxitrichlorids wird der Rückstand mit Eiswasser gegebenenfalls unter Zusatz eines mit Wasser nicht mischbaren Lösungsmittels behandelt. Das aus der getrockneten organischen Phase gegebenenfalls nach Verdampfung des inerten Lösungsmittels isolierte Chlorierungsprodukt ist meist sehr rein und wird anschließend mit Ammoniak in inerten Lösungsmitteln bei 100°C bis 200°C zu den 7-Amino-triazolo[1,5-a]-pyrimidinen umgesetzt. Die Reaktion wird vorzugsweise mit 1- bis 10-molarem Überschuss an Ammoniak unter Druck von 1 bis 100 bar durchgeführt.

Die neuen 7-Amino-azolo[1,5-a]-pyrimidine werden gegebenenfalls nach Verdampfen des Lösungsmittels durch Digerieren in Wasser als kristalline Verbindungen isoliert.

Die β-Ketoester der Formel II können hergestellt werden wie in Organic Synthesis Coll. Vol. 1, S. 248 beschrieben, bzw. sind kommerziell erhältlich.

Alternativ können die neuen Verbindungen der Formel I erhalten werden, indem man substituierte Acylcyanide der Formel VI, in der R¹ und R² die oben angegebenen Bedeutungen haben, mit 3-Amino-1,2,4-triazol der Formel III umsetzt.

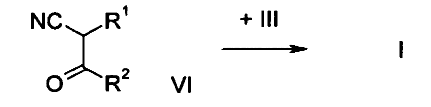

Die Umsetzung kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, aromatische Kohlenwasserstoffe wie Toluol, Benzol oder Mesitylen, Amide wie Dimethylformamid, Diethylformamid, Dibutylformamid, N,N-Dimethylacetamid, niedere Alkansäuren wie Ameisensäure, Essigsäure, Propionsäure oder Basen, wie voranstehend genannt, und Mischungen dieser Lösungsmittel mit Wasser in Frage. Die Umsetzungstemperaturen liegen zwischen 50 und 300°C, vorzugsweise bei 50 bis 150°C, wenn in Lösung gearbeitet wird.

Die neuen 7-Amino-triazolo[1,5-a]-pyrimidine werden gegebenenfalls nach Verdampfen des Lösungsmittels oder Verdünnen mit Wasser als kristalline Verbindungen isoliert.

Die für die Herstellung der 7-Amino-azolo[1,5-a]-pyrimidine benötigten substituierten Alkylcyanide der Formel VI sind teilweise bekannt oder können nach bekannten Methoden aus Alkylcyaniden und Carbonsäureestern mit starken Basen, z.B. Alkalihydriden, Alkalimetallalkoholaten, Alkaliamiden oder Metallalkylen, hergestellt werden (vgl.: J. Amer. Chem. Soc. Bd. 73, (1951) S. 3766).

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder ein- oder zweifach verzweigte Kohlenwasserstoffreste mit 1 bis 4, oder 5 bis 9 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenmethyl: Methylgruppe, in der teilweise oder vollständig die Wasserstoffatome durch Halogenatome wie vorstehend genannt ersetzt sein können: insbesondere Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl;
Cycloalkyl: mono- oder bicyclische, gesättigte Kohlenwasserstoffgruppen mit 3 bis 6 Kohlenstoffringgliedern, wie Cyclopropyl, Cyclobutyl, Cyclopentyl und Cyclohexyl;
Alkoxymethylen und Alkoxyethylen: gesättigte, geradkettige oder ein-, zwei- oder dreifach verzweigte Kohlenwasserstoffkette, die über eine Methylenoxy-, bzw. Ethylenoxygruppe gebunden ist, z. B. Kohlenwasserstoffkette wie voranstehend beschrieben mit 3 bis 11 Kohlenstoffatomen, wie Propoxy-ethyl, Butoxy-ethyl, Pentoxy-ethyl, Hexyloxy-ethyl, Heptyloxy-ethyl, Octyloxy-ethyl, Nonyloxy-ethyl, 3-(3-Ethyl-hexyloxy)-ethyl, 3-(2,4,4-Trimethyl-pentyloxy)-ethyl, 3-(1-Ethyl-3-methyl-butoxy)-ethyl, Ethoxy-propyl, Propoxy-propyl, Butoxy-propyl, Pentoxy-propyl, Hexyloxy-propyl, Heptyloxy-propyl, Octyloxy-propyl, Nonyloxy-propyl, 3-(3-Ethyl-hexyloxy)-propyl, 3-(2,4,4-Trimethylpentyloxy)-propyl, 3-(1-Ethyl-3-methyl-butoxy)-propyl;
In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:

Verbindungen I werden bevorzugt, in denen die Gruppe R¹ maximal 9 Kohlenstoffatome aufweist.

Die Alkylgruppen in R¹ in Formel I stellen bevorzugt unverzweigte oder ein-, zwei- oder dreifach verzweigte Alkylgruppen dar.

In einer Ausgestaltung der erfindungsgemäßen Verbindungen I steht R¹ für C₅-C₉-Alkyl oder C₃-C₁₀-Alkoxyethylen. Für diese Gruppen sind C₄-C₁₀-Alkoxyketten bevorzugt.

Besonders bevorzugt sind Verbindungen I, in denen R¹ für n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl steht.

Daneben sind Verbindungen der Formel I bevorzugt, in denen R¹ für n-Heptyl, 1-Methylhexyl, n-Octyl, 1-Methylheptyl, n-Nonyl, 1-Methyloctyl und 3,5,5-Trimethyl-hexyl steht.

In einer Ausgestaltung der erfindungsgemäßen Verbindungen I steht R² für n-Propyl.

In einer weiteren Ausgestaltung der erfindungsgemäßen Verbindungen I steht R² für n-Butyl.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.
Tabelle 1
Verbindungen der Formel I, in denen R¹ für eine Verbindung jeweils einer Zeile der Tabelle A entspricht und R²n-Propyl bedeutet

Tabelle 2
Verbindungen der Formel I, in denen R¹ für eine Verbindung jeweils einer Zeile der Tabelle A entspricht und R² n-Butyl bedeutet

**Tabelle A**

| **Nr.** | **R¹** |
|---|---|
| A-1 | CH₂CH₂CH₂CH₂CH₃ |
| A-2 | CH(CH₃)CH₂CH₂CH₃ |
| A-3 | CH₂CH(CH₃)CH₂CH₃ |
| A-4 | CH₂CH₂CH(CH₃)CH₃ |
| A-5 | CH₂CH₂CH(CH₃)₂ |
| A-6 | CH(CH₃)CH(CH₃)CH₃ |
| A-7 | CH(CH₃)CH(CH₃)₂ |
| A-8 | CH₂C(CH₃)₃ |
| A-9 | CH₂CH₂CH₂CH₂CH₂CH₃ |
| A-10 | CH(CH₃)CH₂CH₂CH₂CH₃ |
| A-11 | CH₂CH(CH₃)CH₂CH₂CH₃ |
| A-12 | CH₂CH₂CH(CH₃)CH₂CH₃ |
| A-13 | CH₂CH₂CH(CH₃)₂CH₂ |
| A-14 | CH₂CH₂CH₂CH(CH₃)₂ |
| A-15 | CH(CH₃)CH(CH₃)CH₂CH₃ |
| A-16 | CH(CH₃)CH₂CH(CH₃)₂ |
| A-17 | CH₂CH₂C(CH₃)₃ |
| A-18 | CH(CH₃)CH₂CH(CH₃)CH₃ |
| A-19 | CH₂CH₂CH₂CH₂CH₂CH₂CH₃ |
| A-20 | CH(CH₃)CH₂CH₂CH₂CH₂CH₃ |
| A-21 | CH₂CH(CH₃)CH₂CH₂CH₂CH₃ |
| A-22 | CH₂CH₂CH(CH₃)CH₂CH₂CH₃ |
| A-23 | CH₂CH₂CH₂CH(CH₃)CH₂CH₃ |
| A-24 | CH₂CH₂CH₂CH₂CH(CH₃)CH₃ |
| A-25 | CH₂CH₂CH₂CH₂CH(CH₃)₂ |
| A-26 | CH(CH₃)CH(CH₃)CH₂CH₂CH₃ |
| A-27 | CH₂CH(CH₃)CH(CH₃)CH₂CH₃ |
| A-28 | CH₂CH₂CH₂C(CH₃)₃ |
| A-29 | CH(CH₃)CH₂CH(CH₃)CH₂CH₃ |
| A-30 | CH₂CH(CH₃)CH(CH₃)CH₂CH₃ |
| A-31 | CH(CH₃)CH₂CH₂CH(CH₃)CH₃ |
| A-32 | CH₂CH₂CH₂CH₂CH₂CH₂CH₂CH₃ |
| A-33 | CH(CH₃)CH₂CH₂CH₂CH₂CH₂CH₃ |
| A-34 | CH₂CH(CH₃)CH₂CH₂CH₂CH₂CH₃ |
| A-35 | CH₂CH₂CH(CH₃)CH₂CH₂CH₂CH₃ |
| A-36 | CH₂CH₂CH₂CH(CH₃)CH₂CH₂CH₃ |
| A-37 | CH₂CH₂CH₂CH₂CH(CH₃)CH₂CH₃ |
| A-38 | CH₂CH₂CH₂CH₂CH₂CH(CH₃)₂ |
| A-39 | CH₂CH₂CH₂CH₂C(CH₃)₃ |
| A-40 | CH(CH₃)CH(CH₃)CH₂CH₂CH₂CH₃ |
| A-41 | CH₂CH(CH₃)CH(CH₃)CH₂CH₂CH₃ |
| A-42 | CH₂CH₂CH₂C(CH₃)₂CH₂CH₃ |
| A-43 | CH(CH₃)CH₂CH(CH₃)CH₂CH₂CH₃ |
| A-44 | CH₂CH(CH₃)CH(CH₃)CH₂CH₂CH₃ |
| A-45 | CH(CH₃)CH₂CH₂CH(CH₃)CH₂CH₃ |
| A-46 | CH(CH₃)CH₂CH₂CH₂CH(CH₃)₂ |
| A-47 | CH₂CH₂CH₂CH₂CH₂CH₂CH₂CH₂CH₃ |
| A-48 | CH(CH₃)CH₂CH₂CH₂CH₂CH₂CH₂CH₃ |
| A-49 | CH₂CH(CH₃)CH₂CH₂CH₂CH₂CH₂CH₃ |
| A-50 | CH₂CH₂CH(CH₃)CH₂CH₂CH₂CH₂CH₃ |
| A-51 | CH₂CH₂CH₂CH(CH₃)CH₂CH₂CH₂CH₃ |
| A-52 | CH₂CH₂CH₂CH₂CH(CH₃)CH₂CH₂CH₃ |
| A-53 | CH₂CH₂CH₂CH₂CH₂CH₂C(CH₃)₃ |
| A-54 | CH(CH₃)CH(CH₃)CH₂CH₂CH₂CH₂CH₃ |
| A-55 | CH₂CH(CH₃)CH(CH₃)CH₂CH₂CH₂CH₃ |
| A-56 | CH₂CH₂CH₂C(CH₃)₂CH₂CH₂CH₃ |
| A-57 | CH(CH₃)CH₂CH(CH₃)CH₂CH₂CH₂CH₃ |
| A-58 | CH₂CH(CH₃)CH(CH₃)CH₂CH₂CH₂CH₃ |
| A-59 | CH(CH₃)CH₂CH₂CH(CH₃)CH₂CH₂CH₃ |
| A-60 | CH(CH₃)CH₂CH₂CH₂C(CH₃)₃ |
| A-61 | CH₂CH(CH₃)CH₂CH₂C(CH₃)₃ |
| A-62 | CH₂CH₂CH(CH₃)CH₂C(CH₃)₃ |
| A-63 | CH(CH₃)CH₂CH₂CH₂CH₂CH(CH₃)₂ |
| A-64 | CH₂CH(CH₃)CH₂CH₂CH₂CH(CH₃)₂ |
| A-81 | CH₂CH₂-O-CH₂CH₂CH₃ |
| A-82 | CH₂CH₂-O-CH₂CH₂CH₂CH₃ |
| A-83 | CH₂CH₂-O-CH₂CH₂CH₂CH₂CH₃ |
| A-84 | CH₂CH₂-O-CH₂CH₂CH₂CH₂CH₂CH₃ |
| A-85 | CH₂CH₂-O-CH₂CH₂CH₂CH₂CH₂CH₂CH₃ |
| A-86 | CH₂CH₂-O-CH₂CH₂CH₂CH₂CH₂CH₂CH₂CH₃ |
| A-87 | CH₂CH₂-O-CH(CH₃)₂ |
| A-88 | CH₂CH₂-O-C(CH₃)₃ |
| A-89 | CH₂CH₂-O-CH₂C(CH₃)₃ |
| A-90 | CH₂CH₂-O-CH(CH₃)CH₂C(CH₃)₃ |
| A-91 | CH₂CH₂-O-CH(CH₂CH₃)CH₂C(CH₃)₃ |
| A-92 | CH₂CH₂-O-CH₂CH(CH₃)CH₂CH(CH₃)₂ |
| A-93 | CH₂CH₂-O-CH₂CH(CH₂CH₃)CH₂CH₂CH₃ |
| A-94 | CH₂CH₂-O-CH₂CH₂CH(CH₃)CH₂CH(CH₃)₂ |
| A-95 | CH₂CH₂-O-CH₂CH₂CH(CH₃)CH₂C(CH₃)₃ |
| A-96 | CH₂CH₂-O-CH₂CH₂CH(CH₃)CH₂CH₂CH(CH₃)₂ |
| A-97 | CH₂CH₂-O-CH₂CH₂CH(CH₃)CH₂CH₂CH₂CH(CH₃)₂ |

Die Verbindungen I eignen sich als Fungizide. Sie zeichnen sich aus durch eine hervorragende Wirksamkeit gegen ein breites Spektrum von pflanzenpathogenen Pilzen aus der Klasse der *Ascomyceten, Deuteromyceten, Oomyceten* und *Basidiomyceten,* insbesondere aus der Klasse der *Oomyceten.* Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisse, sowie an den Samen dieser Pflanzen.

Speziell eignen sie sich zur Bekämpfung folgender Pflanzenkrankheiten:
- *Altemaria*-Arten an Gemüse und Obst,
- *Bipolaris-* und *Drechslera*-Arten an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Bremia lactucae* an Salat,
- *Erysiphe cichoracearum* und *Sphaerotheca fuliginea* an Kürbisgewächsen,
- *Fusarium-* und *Verticillium*-Arten an verschiedenen Pflanzen,
- *Mycosphaerella*-Arten an Getreide, Bananen und Erdnüssen,
- *Peronospora*-Arten an Kohl und Zwiebelgewächsen,
- *Phakopsora pachyrhizi* und *P. meibomiae* an Soja,
- *Phytophthora infestans* an Kartoffeln und Tomaten,
- *Phytophthora capsici* an Paprika,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Pythium aphanidermatum* an Rasen,
- *Rhizoctonia*-Arten an Baumwolle, Reis und Rasen,
- *Septoria tritici* und *Stagonospora nodorum* an Weizen,
- *Uncinula necator* an Reben,
- *Ustilago*-Arten an Getreide und Zuckerrohr, sowie
- *Venturia*-Arten (Schorf) an Äpfeln und Birnen.

Insbesondere eignen sie sich zur Bekämpfung von Schadpilzen aus der Klasse der *Oomyceten,* wie *Peronospora*-Arten, *Phytophthora*-Arten, *Plasmopara viticola* und *Pseudoperonospora*-Arten.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg, vorzugsweise 5 bis 100 g/100 kg Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

### Beispiele für Formulierungen sind: 1. Produkte zur Verdünnung in Wasser

A Wasserlösliche Konzentrate (SL)
   10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.
B Dispergierbare Konzentrate (DC)
   20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.
C Emulgierbare Konzentrate (EC)
   15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.
D Emulsionen (EW, EO)
   40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.
E Suspensionen (SC, OD)
   20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.
F Wasserdispergierbare und wasserlösliche Granulate (WG, SG)

   50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.
G Wasserdispergierbare und wasserlösliche Pulver (WP, SP) 75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

H Stäube (DP)
   5 Gew.Teile einer erfindungsgemäßen Verbindung werden fein gemahlen und mit 95 % feinteiligem Kaolin innig vermischt. Man erhält dadurch ein Stäubemittel.
I Granulate (GR, FG, GG, MG)
   0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.
J ULV- Lösungen (UL)
   10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyprodinil,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Enilconazol, Epoxiconazol, Fenbuconazol, Fluquiconazol, Flusilazol, Flutriafol, Hexaconazol, Imazalil, lpconazol, Metconazol, Myclobutanil, Penconazol, Propiconazol, Prochloraz, Prothioconazol, Simeconazol, Tebuconazol, Tetraconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Picobenzamid, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel,
- Sonstige Fungizide wie Acibenzolar-S-methyl, Benthiavalicarb, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Phosphorige Säure, Fosetyl, Fosetyl-Aluminium, lprovalicarb, Hexachlorbenzol, Metrafenon, Pencycuron, Propamocarb, Phthalid, Toloclofos-methyl, Quintozene, Zoxamid,
- Strobilurine wie Azoxystrobin, Dimoxystrobin, Enestroburin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von 5-Cyano-dodecan-4-on

Eine Lösung von 0,45 mol Decanitril in 300 ml Tetrahydrofuran (THF) wurde bei -70°C mit einer Lösung von 0,495 mol Butyllithium in Hexan versetzt, dann etwa drei Std. bei dieser Temperatur gerührt und 0,45 mol Ethyl Butanoat zugesetzt. Anschließend wurde noch etwa 16 Std. bei 20 - 25°C gerührt, dann wurden 200 ml Wasser zugesetzt und mit verd. HCL-Lösung angesäuert. Nach Phasentrennung wurde die organische Phase abgetrennt, mit Wasser gewaschen, getrocknet und vom Lösungsmittel befreit. Es blieben 71 g der Titelverbindung zurück.

### Beispiel 2: Herstellung von 7-Amino-5-n-propyl-6-octyl-[1,2,4]-triazolo[1,5-a]-pyrimidin Eine Mischung von je 1,27 mol 5-Cyano-dodecan-3-on aus Beispiel 1, 3-Amino-1,2,4-triazol und 0,25 mol p-Toluolsulfonsäure in 900 ml Mesitylen wurde etwa 4 Std. auf 170°C erhitzt. Nach Abkühlen auf etwa 20 - 25°C wurde der Niederschlag abfiltriert, der dann in Dichlormethan aufgenommen wurde. Aus der Lösung wurde nach Waschen mit Wasser und Trocknen das Lösungsmittel abdestilliert, als Rückstand blieben 102 g der Titelverbindung vom Fp. 165°C zurück.

**Tabelle I - Verbindungen der Formel I**

| Nr. | R¹ | R² | Phys. Daten (Fp. [°C]) |
|---|---|---|---|
| I-1 | CH(CH₃)(CH₂)₅CH₃ | CH₂CH₂CH₃ | 145 |
| I-2 | (CH₂)₇CH₃ | CH₂CH₂CH₃ | 165 |
| I-3 | CH(CH₃)(CH₂)₅CH₃ | CH₂CH₂CH₂CH₃ | 116 |
| I-4 | (CH₂)₇CH₃ | CH₂CH₂CH₂CH₃ | 145 |
| I-5 | (CH₂)₂CH(CH₃)CH₂C(CH₃)₃ | CH₂CH₂CH₃ | 185 |
| I-6 | (CH₂)₅CH₃ | CH₂CH₂CH₃ | 174-175 |
| I-7 | (CH₂)₆CH₃ | CH₂CH₂CH₃ | 169-170 |

### Beispiele für die Wirkung gegen Schadpilze

Die fungizide Wirkung der Verbindungen der Formel I ließ sich durch die folgenden Versuche zeigen:

Die Wirkstoffe wurden als eine Stammlösung aufbereitet mit 25 mg Wirkstoff, welcher mit einem Gemisch aus Aceton und/oder DMSO und dem Emulgator Uniperol® EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

### Beispiel 1 - Wirksamkeit gegen Rebenperonospora verursacht durch Plasmopara viticola

Blätter von Topfreben wurden mit wässriger Suspension in der unten angegebenen Wirkstoffkonzentration bis zur Tropfnässe besprüht. Am folgenden Tag wurden die Unterseiten der Blätter mit einer wässrigen Sporangienaufschwemmung von *Plasmopara viticola* inokuliert. Danach wurden die Reben zunächst für 48 Stunden in einer wasserdampfgesättigten Kammer bei 24°C und anschließend für 5 Tage im Gewächshaus bei Temperaturen zwischen 20 und 30°C aufgestellt. Nach dieser Zeit wurden die Pflanzen zur Beschleunigung des Sporangienträgerausbruchs abermals für 16 Stunden in eine feuchte Kammer gestellt. Dann wurde das Ausmaß der Befallsentwicklung auf den Blattunterseiten visuell ermittelt.

In diesem Test zeigten die mit 250 ppm der Verbindungen I-1, I-2, I-3, I-4, I-6, bzw. I-7 behandelten Pflanzen nicht über 10 % Befall, während die unbehandelten Pflanzen zu 90% befallen waren.

### Beispiel 2: Aktivität gegen die Krautfäule an Tomaten verursacht durch Phytophthora infestans bei protektiver Behandlung

Blätter von getopften Tomatenpflanzen wurden mit einer wässriger Suspension in der unten angegebenen Wirkstoffkonzentration bis zur Tropfnässe besprüht. Am folgenden Tag wurden die Blätter mit einer wässrigen Sporangienaufschwemmung von *Phytophthora infestans* infiziert. Anschließend wurden die Pflanzen in einer wasserdampfgesättigten Kammer bei Temperaturen zwischen 18 und 20°C aufgestellt. Nach 6 Tagen hatte sich die Krautfäule auf den unbehandelten, jedoch infizierten Kontrollpflanzen so stark entwickelt, dass der Befall visuell in % ermittelt werden konnte.

In diesem Test zeigten die mit 250 ppm der Verbindungen I-1, I-2, I-5, I-6, bzw. I-7 behandelten Pflanzen maximal 3 % Befall, während die unbehandelten Pflanzen zu 95% befallen waren.

## Patentansprüche

1. Triazolopyrimidine der Formel I 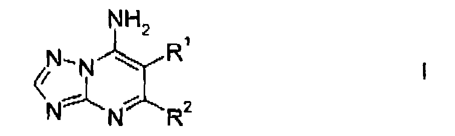 in der die Substituenten folgende Bedeutung haben:
R¹ C₅-C₉-Alkyl oder C₃-C₁₀-Alkoxyethylen;
R² n-Propyl oder n-Butyl.

2. Verbindungen der Formel I gemäß Anspruch 1, worin R¹ für eine unsubstituierte unverzweigte oder ein-, zwei- oder dreifach verzweigte Alkylkette mit bis zu 9 Kohlenstoffatomen steht.

3. Verbindungen der Formel I gemäß Anspruch 1 oder 2, worin R² für n-Propyl steht.

4. Verbindungen der Formel I gemäß Anspruch 1 oder 2, worin R² für n-Butyl steht.

5. 6-(1-Methyf-heptyl)-5-propyl-[1,2,4]triazolo[1,5-a]pyrimidin-7-ylamin;
6-Octyl-5-propyl-[1,2,4]triazolo[1,5-a]pyrimidin-7-ylamin;
5-Butyl-6-(1-methyl-heptyl)-[1,2,4]triazolo[1,5-a]pyrimidin-7-ylamin;
5-Butyl-6-octyl-[1,2,4]triazolo[1,5-a]pyrimidin-7-ylamin;
5-Propyl-6-(3,5,5-trimethyl-hexyl)-[1,2,4]triazolo[1,5-a]pyrimidin-7-ylamin;
6-Hexyl-6-propyt-[1,2,4]triazolo[1,5-a]pyrimidin-7-ylamin;
6-Heptyl-5-propyl-[1,2,4]triazolo[1,5-a]pyrimidin-7-ylamin.

6. Verfahren zur Herstellung von Verbindungen der Formel I gemäß einem der Ansprüche 1 bis 5, **dadurch gekennzeichnet, dass** man β-Ketoester der Formel II, 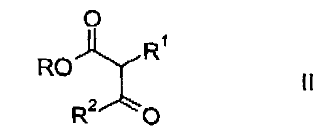 in der R für C₁-C₄-Alkyl steht, mit 3-Amino-1,2,4-triazol der Formel III 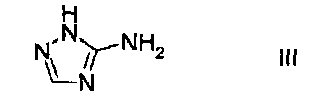 zu 7-Hydroxytriazolopyrimidinen der Formel IV 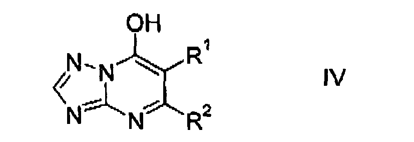 umsetzt, welche zu Verbindungen der Formel V, 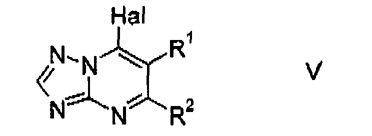 in der Hal für Chlor oder Brom steht, halogeniert werden, und V mit Ammoniak umgesetzt wird.

7. Verfahren zur Herstellung von Verbindungen der Formel I gemäß einem der Ansprüche 1 bis 5, **dadurch gekennzeichnet, dass** man Acylcyanide der Formel VI, 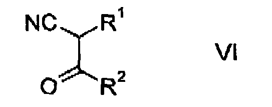 mit 3-Amino-1,2,4-triazol der Formel III gemäß Anspruch 6 umsetzt.

8. Fungizides Mittel, enthaltend einen festen oder flüssigen Träger und eine Verbindung der Formel I gemäß einem der Ansprüche 1 bis 5.

9. Saatgut, enthaltend eine Verbindung der Formel I gemäß einem der Ansprüche 1 bis 5 in Mengen von 1 bis 1000 g pro 100 kg.

10. Verfahren zur Bekämpfung von pflanzenpathogenen Schadpilzen, **dadurch gekennzeichnet, dass** man die Pilze, oder die vor Pilzbefall zu schützenden Materialien, Pflanzen, den Boden oder Saatgüter mit einer wirksamen Menge einer Verbindung der Formel I gemäß einem der Ansprüche 1 bis 5 behandelt.

## Claims

1. A triazolopyrimidine of the formula I 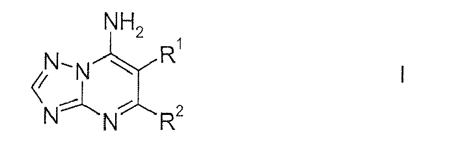 in which the substituents are as defined below:
R¹ is C₅-C₉-alkyl or C₃-C₁₀-alkoxyethylene;
R² is n-propyl or n-butyl.

2. The compound of the formula I according to claim 1 in which R¹ is an unsubstituted straight-chain or mono-, di- or tribranched alkyl chain having up to 9 carbon atoms.

3. The compound of the formula I according to claim 1 or 2 in which R² is n-propyl.

4. The compound of the formula I according to claim 1 or 2 in which R² is n-butyl.

5. 6-(1-Methylheptyl)-5-propyl-[1,2,4]triazolo[1,5-a]pyrimidin-7-ylamine;
6-octyl-5-propyl[1,2,4]triazolo[1,5-a]pyrimidin-7-ylamine;
5-butyl-6-(1-methylheptyl)[1,2,4]triazolo[1,5-a]pyrimidin-7-ylamine;
5-butyl-6-octyl[1,2,4]triazolo[1,5-a]pyrimidin-7-ylamine;
5-propyl-6-(3,5,5-trimethylhexyl)[1,2,4]triazolo[1,5-a]pyrimidin-7-ylamine;
6-hexyl-5-propyl[1,2,4]triazolo[1,5-a]pyrimidin-7-ylamine;
6-heptyl-5-propyl[1,2,4]triazolo[1,5-a]pyrimidin-7-ylamine.

6. A process for preparing compounds of the formula I according to any of claims 1 to 5, wherein β-ketoesters of the formula II, 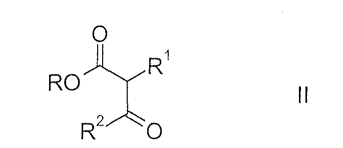 in which R is C₁-C₄-alkyl are reacted with 3-amino-1,2,4-triazole of the formula III 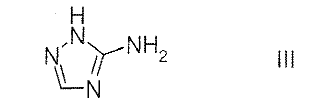 to give 7-hydroxytriazolopyrimidines of the formula IV 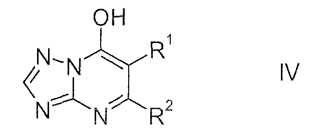 which are halogenated to give compounds of the formula V 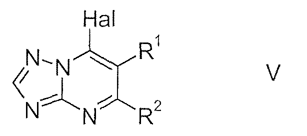 in which Hal is chlorine or bromine and V is reacted with ammonia.

7. A process for preparing compounds of the formula I according to any of claims 1 to 5, wherein acylcyanides of the formula VI, 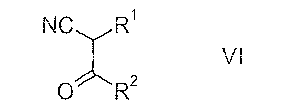 are reacted with 3-amino-1,2,4-triazole of the formula III according to claim 6.

8. A fungicidal composition comprising a solid or liquid carrier and a compound of the formula I according to any of claims 1 to 5.

9. Seed comprising a compound of the formula I according to any of claims 1 to 5 in amounts of 1 to 1000 g per 100 kg.

10. A method for controlling phytopathogenic harmful fungi wherein the fungi or the materials, plants, the soil or seed to be protected against fungal attack are treated with an effective amount of a compound of the formula I according to any of claims 1 to 5.

## Revendications

1. Triazolopyrimidines de formule I : 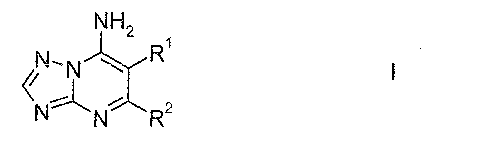 dans laquelle les substituants ont la signification suivante :
R¹ représente un alkyle en C₅-C₉ ou un alcoxy(C₃-C₁₀)éthylène, et
R²représente le n-propyle ou le n-butyle.

2. Composés de formule 1 selon la revendication 1, dans lequel R¹ représente une chaîne alkyle non substituée, non ramifiée ou ramifiée une, deux ou trois fois, ayant jusqu'à 9 atomes de carbone.

3. Composés de formule I selon la revendication 1 ou 2, dans lequel R² est le n-propyle.

4. Composés de formule I selon la revendication 1 ou 2, dans lequel R² est le n-butyle.

5. 6-(1-méthyl-heptyl)-5-propyl-[1,2,4]triazolo[1,5-a]-pyrimidin-7-ylamine;
6-octyl-5-propyl-[1,2,4]triazolo[1,5-a]pyrimidin-7-yl-amine;
5-butyl-6-(1-méthyl-heptyl)-[1,2,4]triazolo[1,5-a]-pyrimidin-7-ylamine;
5-butyl-6-octyl-[1,2,4]triazolo[1,5-a]pyrimidin-7-yl-amine;
5-propyl-6-(3,5,5-triméthyl-hexyl)-[1,2,4]triazolo[1,5-a]pyrimidin-7-ylamine;
6-hexyl-5-propyl-[1,2,4]triazolo[1,5-a]pyrimidin-7-yl-amine;
6-heptyl-5-propyl-[1,2,4]triazolo[1,5-a]pyrimidin-7-yl-amine.

6. Procédé pour fabriquer des composés de formule I selon l'une quelconque des revendications 1 à 5, **caractérisé en ce que** l'on fait réagir des β-cétoesters de formule II : 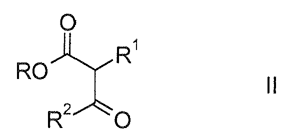 dans laquelle R représente un alkyle en C₁-C₄ avec du 3-amino-1,2,4-triazole de formule III : 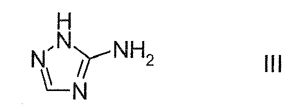 pour donner des 7-hydroxytriazolopyrimidines de formule IV : 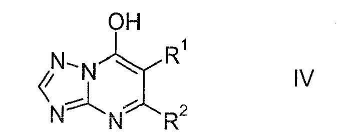 qui sont halogénées pour former des composés de formule V : 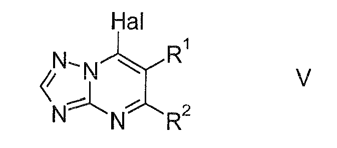 dans laquelle Hal représente le chlore ou le brome, et l'on fait réagir V avec de l'ammoniac.

7. Procédé pour fabriquer des composés de formule I selon l'une quelconque des revendications 1 à 5, **caractérisé en ce que** l'on fait réagir des cyanures d'acyle de formule VI : 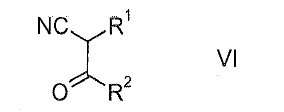 avec du 3-amino-1,2,4-triazole de formule III selon la revendication 6.

8. Agent fongicide contenant un véhicule solide ou liquide et un composé de formule I selon l'une quelconque des revendications 1 à 5.

9. Semences contenant un composé de formule I selon l'une quelconque des revendications 1 à 5 en quantités de 1 à 1000 g/100 kg.

10. Procédé pour lutter contre les champignons nuisibles phytopathogènes, **caractérisé en ce que** l'on traite les champignons, les matériaux, les plantes, le sol ou les semences à protéger de l'infection par les champignons avec une quantité efficace d'un composé de formule I selon l'une quelconque des revendications 1 à 5.
